# EUROPÄISCHE PATENTSCHRIFT

(11) **EP 1 198 209 B1**
(45) Veröffentlichungstag und Bekanntmachung des Hinweises auf die Patenterteilung: **11.05.2005**
(21) Anmeldenummer: 00962288.7
(22) Anmeldetag: 02.08.2000
(51) Int. Cl.: A61F 2/44

(54) **ZWISCHENWIRBEL-NUCLEUS-PROTHESE**
INTERVERTEBRAL NUCLEUS PROSTHESIS
PROTHESE A NOYAU INTERVERTEBRAL

(30) Priorität: 03.08.1999 FR 9910167
(43) Veröffentlichungstag der Anmeldung: 24.04.2002
(60) Teilanmeldung: 05075508.1
(73) Patentinhaber: LDR Medical SAS, 10430 Rosieres-Pres-Troyes (FR)
(72) Erfinder: Gau, Michel, 11590 Ouveillan (FR)
(74) Vertreter: Debay, Yves
(86) Internationale Anmeldenummer: PCT/EP2000/007494
(87) Internationale Veröffentlichungsnummer: WO 2001/008612

(56) Entgegenhaltungen:
- EP-A- 0 577 179
- EP-A- 0 621 020
- DE-C- 19 527 975
- DE-U- 9 304 368

## Beschreibung

Die Erfindung betrifft eine Zwischenwirbel-Nucleus-Prothese, deren Aufgabe es ist, die Beweglichkeit im Bereich einer Bandscheibe wieder herzustellen. Diese Beweglichkeit wird physiologisch durch den Nucleus erzeugt, der als mechanisches Gelenkelement der Wirbelkörper angesehen werden kann.

Physiologisch wird der Nucleus von einem im wesentlichen sphärischen, nicht dehnbaren, jedoch verformbaren Körper gebildet, der wie eine Kugel zwischen zwei Wirbelendflächen eingesetzt ist, und zwar im wesentlichen in deren Zentrum, und der Bewegungen in Form der Neigung, der Rotation und der Verschiebung ermöglicht.

Die Wirbelendflächen werden ihrerseits von konzentrischen Faserlagen umgeben, die als Annulus bezeichnet werden und die Fasern enthalten, die von einer Schicht zur anderen sich schräg überkreuzen.

In dieser Anordnung bewegt sich der Nucleus, der eigentlich eine verformbare, jedoch nicht dehnbare Kapsel darstellt, die mit einer hydrophilen, geleeartigen Substanz gefüllt ist (Mucopolysaccharide) bei der Beugung der Wirbelsäule nach vorne oder nach hinten selbst nach hinten beziehungsweise nach vorn, wobei diese Bewegung des Nucleus durch die hinteren und die vorderen Fasern des Annulus und durch unterschiedliche Längsbänder begrenzt wird.

Bei einer seitlichen Neigung der Wirbelsäule dehnt die Verlagerung des Nucleus die Fasern auf der konvexen Seite. Die Verlagerung wird dabei durch die Zwischenwirbelbänder begrenzt.

Bei einer Drehung der Wirbelsäule wird die Bandscheibe mit Scherkräften beaufschlagt.

Der Nucleus, der einen hydrophilen Körper darstellt, hat im Ruhezustand einen bestimmten osmotischen Druck. Bei Belastung verliert der Nucleus Wasser. Die Dicke der Bandscheibe nimmt ab, sobald der Druck wieder reduziert wird, tritt Rehydrierung ein.

Die pathologischen Zustände, die die wechselseitigen Bewegungen der verschiedenen Wirbelkörper stören, sind dabei im wesentlichen Überlastung oder zu starke und zu häufige Belastungen oder natürliche Alterungsprozesse.

Bei einer zu starken, zu häufigen oder zu lang anhaltenden Belastung tritt die Rehydrierung nicht ein und damit kann der Druck, der vom Nucleus auf die die verschiedenen konzentrischen Lagen des Annulus bildenden Fasern ausübt, nicht mehr aufgebaut werden, damit spielt auch dieser Druck nicht mehr die Rolle der Rückstellung bei den Bewegungen. In diesem Falle tritt eine Instabilität auf und eine begleitende Arthrose.

Es ist Aufgabe der Erfindung, die Entwicklung dieser Instabilität dadurch zu verhindern, daß eine Nucleus-Prothese eingesetzt wird, deren Implantation in vivo erfolgen kann und mit weniger Traumatisierung als dies bei bekannten Prothesen dieser Art bisher möglich war.

Diese bekannten Prothesen, deren Prinzipien nachstehend untersucht werden, zeigen Nachteile, die man verstehen kann, wenn man die gegenseitigen Bewegungen und die Rolle des Nucleus bei diesen Bewegungen kennt.

Diese Nachteile treten auf sowohl bei der Benutzung als auch beim Einsetzen dieser bekannten Implantate.

Zunächst ist einfach vorgeschlagen worden, die Bandscheiben zu entfernen, die sich in dem traumatisierten Bereich befinden. Dann hat man einen knöchernen Pfropfen eingesetzt, teilweise mit und teilweise ohne Osteosynthese.

Die dadurch erzielte Blockierung von zwei benachbarten Wirbelkörpern hat jedoch nur die Belastungen auf die Bandscheiben der unmittelbar benachbarten Wirbelkörper übertragen, die dadurch übermäßig belastet und schnell geschädigt wurden, das Problem ist also nur verlagert und nicht gelöst worden.

Man hat weiterhin eine vollständige Bandscheibenprothese vorgeschlagen, die sowohl Annulus als auch Nucleus umfaßt.

Diese Praxis hat den Hauptnachteil, daß dazu eine vollständige Entfernung der geschädigten Bandscheibe notwendig ist und daß die Totalprothese dann zwischen zwei benachbarte Wirbelkörper eingeführt werden muß.

Für beide Phasen dieses Eingriffes muß man die gemeinsamen Wirbelkörperlängsbänder durchschneiden, nämlich das vordere Längsband (LVCA) und das hintere Längsband (LVCP), so daß man die natürlichen Bewegungen der Wirbelsäule nicht wieder herstellen kann, diese büßt somit an Zuverlässigkeit ein.

Um diese Nachteile zu vermeiden, wird im Rahmen der vorliegenden Erfindung davon ausgegangen, daß eine Nucleus-Prothese eingesetzt wird, ohne daß man die gesamte Bandscheibe reseziert. Auf diese Weise läßt sich durch das Ersetzen des Nucleus bereits die Entwicklung der Instabilität begrenzen, die ursächlich für die Verschlechterung ist.

Die Nucleus-Prothese erlaubt ohne Verletzung des Annulus oder der Längsbänder (LVCA und LVCP) durch die Verschiebung des ersetzten Nucleus auf den Annulus Druck auszuüben und ihn daher bei der Bewegung der Wirbelsäule so zu spannen, daß er die Gleichgewichtslage wiederherstellt.

Das Rotationszentrum, das auf diese Weise wiederhergestellt worden ist, bleibt tatsächlich beweglich und in der Lage, sich an die verschiedenen Beugebewegungen nach vorne und nach hinten, an Streckbewegungen und seitliche Neigungsbewegungen anzupassen.

Das Dokument EP 0 621 020 offenbart eine Zwischenwirbel-Nucleus-Prothese als Ersatz für einen Teil des kernbereichs einer Bandscheibe, geeignet zur Übertragung von Druckkräften, die aus mindestens drei beweglichen, insbesondere Kugelförmigen, körpern aus einem nicht oxidierenden Material besteht, deren Volumen an das des biologischen Nucleus angepaßt ist.

Die Nucleus-Prothese der vorliegenden Erfindung besteht aus einer oder mehreren Kugeln, die hart, glatt und nicht oxidierend sind und die frei beweglich im Inneren eines festen Käfigs angeordnet sind, der seinerseits nicht oxidierend ist. Diese Prothese kann in dem Volumen untergebracht werden, welches nach der Entfernung des Nucleus zwischen zwei Wirbelendflächen frei wird.

Die Figur 1 zeigt eine Draufsicht auf einen Wirbelkörper mit den jeweiligen Positionen des Nucleus, des Annulus und der vorderen und hinteren Längsbänder.

Die Figur 2 ist eine perspektivische Ansicht eines Wirbelkörpers und zeigt den Annulus und den von ihm umgebenen Nucleus, der sich am Wirbelkörper abstützt.

Die Figur 3 ist eine perspektivische schematische Ansicht eines diametral geschnittenen Annulus und zeigt die konzentrischen, sich kreuzenden Faserlagen.

Die Figuren 4 und 5 sind schematische Seitenansichten von zwei benachbarten Wirbelkörpern bei einer Beugebewegung nach vorne beziehungsweise hinten.

Die Figur 6 ist eine schematische Vorderansicht von zwei benachbarten Wirbelkörpern während einer seitlichen Beugung der Wirbelsäule.

Die Figur 7 ist eine Draufsicht auf einen Wirbelkörper bei einer Rotationsbewegung.

Die Figuren 8, 9 und 10 sind schematische Ansichten von der Seite, von oben und von vorne einer Nucleus-Prothese mit nur einer Kugel.

Die Figur 11 ist eine schematische, beispielshafte Längsschnittansicht längs Linie A-A in Figur 8.

Die Figuren 12, 13 und 14 sind schematische Ansichten von oben, von vorne und von der Seite einer Nucleus-Prothese mit mehreren Kugeln.

Die Figuren 15, 16 und 17 sind schematische Ansichten von oben, von vorne und von der Seite einer Nucleus-Prothese mit einer Dämpfungseinrichtung.

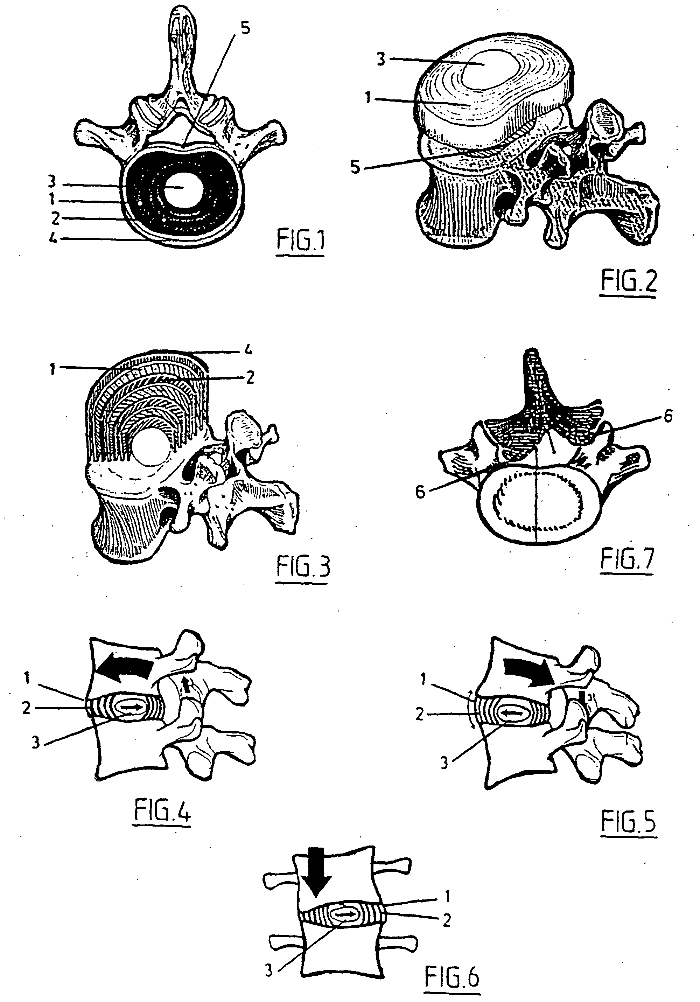

Die Figuren 1 und 2 zeigen gut eine Zwischenwirbelebene, die gebildet wird durch eine Vielzahl von konzentrischen Lagen von sich kreuzenden Fasern 1 und 2, deutlich sichtbar in Figur 3, und in deren Zentrum jeweils der Nucleus 3 angeordnet ist, während an der Außenseite das vordere Längsband (LVCA) 4 und das hintere Längsband (LVCP) 5 angeordnet sind.

Die Figuren 4 und 5 zeigen die Kompressionskräfte, die durch den Nucleus 3 auf die konzentrischen Annuluslagen ausgeübt werden, und zwar jeweils an der Außenseite des Biegewinkels bei der Biegung nach vorne oder nach hinten.

Der Nucleus 3 übt in gleicher Weise einen Druck auf die Ringe des Annulus aus, und zwar auf der Außenseite des seitlichen Neigungswinkels (Figur 6).

Jede dieser Verschiebungen des Nucleus 3 stellt eine Rückkehr in die Gleichgewichtslage sicher.

Bei einer Rotation in einer horizontalen Ebene sind es die Gelenkkapseln 6, die längs eines fiktiven Kreises an der Außenseite liegen, die die kreisförmige Verlagerung ermöglichen und die die Rotationsbewegung begrenzen (Figur 7), der Nucleus 3 wird dabei mit Scherkräften beaufschlagt.

Der Nucleus spielt also in all diesen Fällen die Rolle eines Bewegungsdämpfers und stellt bei dieser'Bewegung die Rückkehr in die Gleichgewichtslage sicher. Man erkennt daraus die Folgen, die sich einstellen, wenn der Nucleus verändert wird.

Man erkennt daraus auch, daß eine Wiederherstellung der gegenseitigen Bewegungen benachbarter Wirbelkörper bereits dadurch erreicht werden kann, daß man eine Nucleus-Prothese einsetzt, deren Implantation einen sehr großen chirurgischen Vorteil bedeutet.

Bei den bestehenden operativen Techniken führt die chirurgische Wirbelkörperverschmelzung zu einer dauerhaften Fusion von benachbarten Wirbeln, die zu beiden Seiten der geschädigten Bandscheibe angeordnet sind. Zu diesem Zweck wird die Bandscheibe vollständig entfernt.

Wenn die Fusion von zwei Wirbelkörpern durch Arthrodese gelingt, dann werden die Probleme der Degenerierung auf die benachbarten Bandscheiben (oben oder unten) übertragen, wobei diese Probleme noch durch die Festigkeit des auf diese Weise erzeugten Wirbelblocks erhöht werden.

Wenn jedoch die Fusion nicht gelingt, was der Grund für häufige Fehlschläge ist und was zu einer Fortdauer der Beschwerden führt, dann kann man häufig nur sehr schwierig nachoperieren.

Im Fall der Implantation einer Totalprothese der Bandscheibe hat man festgestellt, daß diese ein kompliziertes und schwieriges chirurgisches Verfahren notwendig macht, wobei man eine große Öffnung benötigt, um die natürliche Bandscheibe entfernen und die Totalprothese einsetzen zu können.

Dazu gehört die Notwendigkeit der Durchtrennung und der Verlagerung großer Bauchgefäße (Aorta und Vene) und des Nervenbündels der Sexualorgane.

Dabei ergibt sich das Risiko großer Gefäßblutungen, die nur schwer zu kontrollieren sind.

Dies kann auch zu schwerwiegenden Sexualproblemen führen: Impotenz und retrograde Ejakulation.

Darüber hinaus führt die Totalresektion der Bandscheibe zu einer Zerstörung des gesamten Annulus und der vorderen und hinteren Zwischenwirbellängsbänder 4 und 5 und erzeugt dadurch schwerwiegende Instabilitätsrisiken.

Außerdem gibt es in diesem Falle keinerlei Rückkehr, es bleibt nur der Weg einer Wirbelfusion mit den oben beschriebenen Nachteilen.

Die erfindungsgemäße Nucleus-Prothese weist keinen dieser Nachteile auf. Sie ermöglicht es, den geschädigten Nucleus sowohl der Form als auch der Funktion nach zu ersetzen. Sie ist geeignet, die Degeneration der Bandscheibe dadurch zu beenden, daß sie diese wieder stabilisiert.

Das chirurgische Implantationsverfahren ist außerordentlich einfach.

Zunächst kann die Implantation endoskopisch durchgeführt werden. Sie ist schnell und schonend.

Im Falle eines Fehlschlages kann man durch Entfernen der Nucleus-Prothese den ursprünglichen Zustand wieder herstellen.

Sie ist nicht mit Gefäßrisiken verbunden und auch nicht mit Risiken für die Sexualorgane.

Es genügt tatsächlich, endoskopisch eine Öffnung zwischen zwei Wirbelkörper durch den Annulus hindurch einzubringen, die gerade groß genug ist, um den geschädigten Nucleus zu erreichen und zu entfernen und um anschließend auf demselben Wege den künstlichen Nucleus einzuführen, der sich automatisch in der ursprünglichen Kammer zentriert, anschließend wird die erzeugte Öffnung durch eine Naht verschlossen.

Unabhängig davon, daß dieser Eingriff schnell erfolgt, kann der Faserkörper, der nur geringfügig durch den Einschnitt geschädigt ist, sich spontan und schnell und praktisch schmerzfrei erholen. Die vorderen und hinteren Längsbänder werden in keiner Weise traumatisiert, so daß man die ursprüngliche Beweglichkeit der Wirbelsäule wiederherstellen kann.

Die einfache Implantation dieser Nucleus-Prothese und die Tatsache, daß bei diesem Eingriff keinerlei Risiken auftreten, führt dazu, daß ihre Anwendung immer dann angezeigt ist, wenn primäre oder sekundäre Bandscheibenschädigungen eingetreten sind, und zwar bereits beim ersten Auftreten der Krankheit, bevor definitive Schädigungen des Knochens und der Gelenke auftreten.

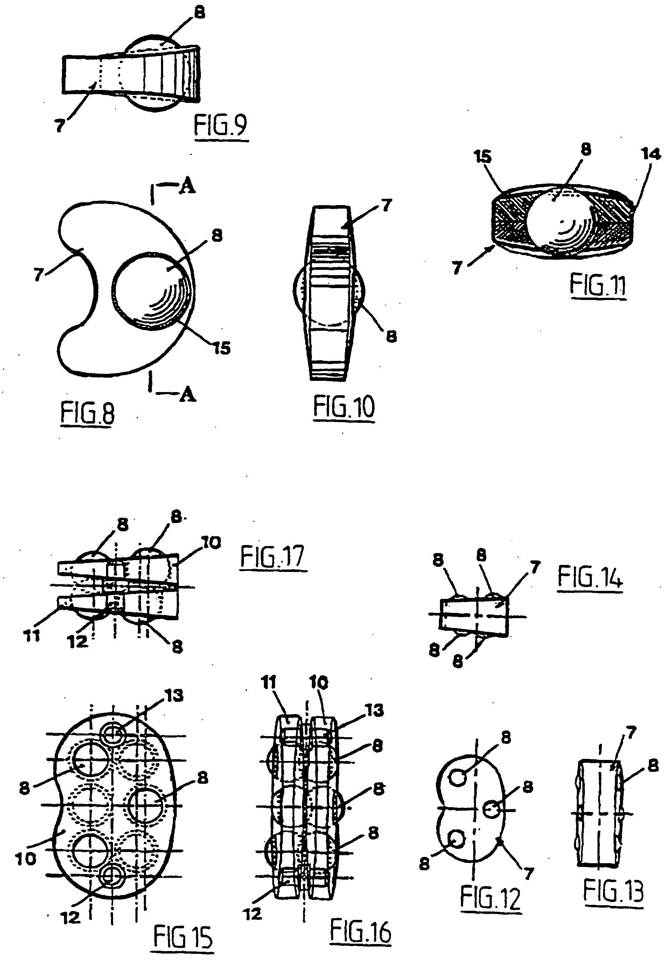

Die Nucleus-Prothese besteht im wesentlichen aus mindestens einem beweglichen Körper 8, aus einem festen, nicht oxidierenden, biokompatiblen Material, welcher sich in zwei Achsen einer Ebene bewegen kann (beispielsweise eine nichtrostende Stahlkugel oder eine Kugel aus Titan), deren Volumen angepaßt ist an das des biologischen Nucleus. Dieser bewegliche Körper oder diese Kugel 8 ist in einem im folgenden als Käfig bezeichneten Körper 7 gelagert, der ein Gehäuse 14 umfaßt (beispielshaft in Figur 11 dargestellt), welches aus einem leichten, festen, nicht oxidierenden und biokompatiblen Material besteht (beispielsweise Titan), wobei dieses Gehäuse 14 eine Masse 15 enthält aus einem Material mit einem minimalen Reibungskoeffizienten (beispielsweise Polyethylen). Im Inneren befindet sich ein Aufnahmeraum für den beweglichen Körper oder die Kugel 8, diese ist in diesem Aufnahmeraum gefangen, jedoch frei um ihren Mittelpunkt verdrehbar und derart gelagert, daß sie auf beiden gegenüberliegenden Seiten (Oberseite und Unterseite) des Käfigs 7 in Form einer Kugelkalotte austritt, deren Höhe etwa 1/10 des Durchmessers des beweglichen Körpers oder der Kugel 8 entspricht, wobei dieser Wert nicht streng eingehalten werden muß.

Das Volumen des Käfigs 7, der den mobilen Körper 8 aufnimmt, ist seinerseits möglichst eng an das Volumen des biologischen Nucleus angepaßt, wobei natürlich berücksichtigt ist, daß der Käfig 7 als Lagerung für die Kugel 8 dienen muß. Dadurch kann sich die Prothese selbst positionieren, es ist ihr also möglich, sich immer in der anatomischen Position zu befinden und dadurch die natürliche Bewegung zwischen zwei Wirbelkörpern wiederherzustellen.

Der die Kugel 8 lagernde Käfig 7 kann in der Draufsicht (Figur 8) eine mehr oder weniger gebogene Form aufweisen, die symmetrisch im Bezug auf eine transversale Mittelebene ist. Im Querschnitt (Figur 10) weist der Käfig vorzugsweise die Form eines Trapezes auf, dessen kleine Stirnfläche die Enden der mehr oder weniger stark gebogenen Form aufnimmt.

Diese trapezförmige, von vorne nach hinten orientierte Asymmetrie des Käfigs 7 führt dazu, daß eine Drehung desselben zwischen den zwei Wirbelkörpern vermieden wird, wenn sich die in dieser Weise gebildete, den künstlichen Nucleus (Kugel 8) aufnehmende Prothese verschiebt.

Bei einer in den Figuren 12, 13 und 14 dargestellten abgewandelten Ausführungsform kann der Käfig 7, der im Querschnitt im wesentlichen die Form eines gleichschenkligen Trapezes aufweist (Figur 13), mehrere identische Kugeln 8 (Figur 14) aufnehmen, die an der Außenseite eine gemeinsame fiktive Ebene berühren, die auf beiden Seiten der horizontalen Mittelebene des Käfigs angeordnet sind. Die Kugeln befinden sich auf beiden Seiten des Käfigs 7 an den drei Eckpunkten eines gleichschenkligen Dreiecks.

Um den Komfort zu erhöhen, kann der Käfig 7 entsprechend den Ausführungsformen der Figuren 15, 16 und 17 aus zwei identischen Einzelkörpern 10, 11 bestehen, die fest, nicht oxidierbar und biokompatibel ausgebildet sind und die Form eines gleichschenkligen Trapezes aufweisen. Sie sind so angeordnet, daß ihre senkrecht auf den parallelen Basisflächen stehenden, längs deren Mittellinien verlaufenden Mittelebenen im wesentlichen parallel zueinander verlaufen und daß die großen Basisflächen der trapezförmigen Einzelkörper 10 und 11 im wesentlichen in derselben senkrechten Ebene liegen.

Die zwei auf diese Weise ausgebildeten Einzelkörper sind untereinander durch zwei elastische Lagerelemente 12 und 13 verbunden, die senkrecht zu den Mittelebenen der Einzelkörper 10 und 11 verlaufen und nahe dem Ende einer großen Achse angeordnet sind, die ihrerseits eine gemeinsame Tangente an die Sätze der Kugeln 8 ausbildet, die in jeder Außenseite der Gesamtanordnung so angeordnet sind, daß die Kugelh an den drei Ecken eines gleichschenkligen Dreiecks angeordnet sind, die ihrerseits auf gegenüberliegenden Seiten der Anordnung in entgegengesetzte Richtung weisen.

Wenn eine Prothese entsprechend den Figuren 8 bis 17 eingesetzt ist, dann ist verständlich, daß bereits unmittelbar nach dem Einführen in den Hohlraum des Nucleus die Prothese die Rolle des biologischen Nucleus übernimmt und die sich kreuzenden Fasern des Annulus bei einer seitlichen oder bei einer Bewegung nach vorne oder nach hinten unter Spannung setzt und dadurch eine Rückkehr in die Gleichgewichtslage erzeugt. Der schnelle Eingriff, der zum Einsetzen notwendig ist, vermeidet jegliche Traumatisierungsrisiken und führt zu einer schnellen Heilung der einzig notwendigen Öffnung.

Die endgültige Auswahl der hier vorgeschlagenen Modelle, die alle dieselben Vorteile hinsichtlich des Einsetzens und hinsichtlich der physiologischen Resultate aufweisen, wird sich aufgrund der klinischen Resultate im Laufe der Zeit ergeben.

Sebstverständlich kann der Käfig 7, der eine Kugel 8 oder mehrere Kugeln 8 enthält und für den vorstehend mehrere Ausführungsformen vorgeschlagen worden sind, ohne Abkehr von den erfindungsgemäßen Gedanken auch andere äquivalente Formen aufweisen, es ist lediglich wichtig, daß er die Kugel oder die Kugeln 8 lagert, daß diese in ihm frei drehbar sind und daß sein Volumen an das Volumen angepaßt ist, welches nach Entfernung des biologischen Nucleus zur Verfügung steht.

Die beschriebene Nucleus-Prothese kann bei allen vorstehend beschriebenen Ausführungsformen spontan in der anatomischen Umgebung die eigene Position einnehmen, so daß alle natürlichen Bewegungen zwischen zwei Wirbelkörpern wiederhergestellt werden können (seitliche Neigung, Neigung nach vorne und nach hinten, Rotation). Die konische Form (im Querschnitt trapezförmig) des Käfigs 7 erleichtert die Verschiebung in der Ebene der Wirbelendflächen und verhindert gleichzeitig die Rotation des Käfigs um seine zentrale Achse, außerdem das Einsinken des Implantates in die Wirbelendflächen.

## Patentansprüche

1. Zwischenwirbel-Nucleus-Prothese, deren Volumen angepaßt ist an das des biologischen Nucleus, **dadurch gekennzeichnet, daß** sie aus mindestens einem in zwei Richtungen einer Ebene beweglichen kugelförmigen Körper (8) aus einem festen, nicht oxidierenden, biokompatiblen Material besteht, der in einem Käfig (7) um seinen Mittelpunkt frei drehbar und unverschiebbar gelagert ist, und der an beiden gegenüberliegenden Seiten des Käfigs in Form einer Kugelkalotte hervorsteht.

2. Prothese nach Anspruch 1, **dadurch gekennzeichnet, daß** die gegenüberliegenden Seiten des Käfigs (7) gebogen ausgebildet und symmetrisch bezüglich einer Quermittelebene sind.

3. Prothese nach einem der Ansprüche 1 oder 2, **dadurch gekennzeichnet, daß** der Käfig (7) ein Gehäuse (14) aus leichtem, festem, nicht oxidierendem, biokompatiblem Material umfaßt, insbesondere aus Titan, und eine Masse (15) aus einem Material mit minimalem Reibungskoeffizienten enthält, insbesondere Polyethylen, und daß im Inneren der Masse ein Aufnahmeraum für den beweglichen kugelförmigen Körper (8) vorgesehen ist, in dem dieser frei drehbar gefangen gelagert ist.

4. Prothese nach einem der Ansprüche 1 bis 3, **dadurch gekennzeichnet, daß** der Käfig (7) im wesentlichen die Form eines gleichschenkligen Trapezes aufweist und mehrere gleiche Kugeln (8) lagert, die auf beiden Seiten der horizontalen Mittelebene des Käfigs jeweils eine gleiche fiktive außerhalb des Käfigs liegende Ebene berühren und die auf beiden Seiten des Käfigs (7) an den Eckpunkten eines gleichschenkligen Dreiecks liegen.

5. Prothese nach einem der voranstehenden Ansprüche, **dadurch gekennzeichnet, daß** die Höhe der aus dem Käfig hervorstehenden Kugelkalotte etwa jeweils ein Zehntel des Durchmessers des beweglichen Körpers (8) beträgt.

6. Zwischenwirbel-Nucleus-Prothese, deren Volumen angepaßt ist an das des biologischen Nucleus, **dadurch gekennzeichnet, daß** sie einen Käfig (7) besitzt, der aus zwei identischen Einzelkörpern besteht, wobei mehrere in zwei Richtungen einer Ebene bewegliche kugelförmige Körper (8) aus einem festen, nicht oxidierenden, biokompatiblen Material in jedem Einzelkörper um ihren Mittelpunkt frei drehbar und unverschiebbar gelagert sind und an beiden gegenüberliegenden Seiten des Einzelkörper (10) in Form einer Kugelkalotte hervorstehen, wohin die zwei identischen Einzelkörper (10,11) gleichschenklig trapezförmig und so angeordnet sind, daß ihre senkrecht auf den parallelen Basisflächen stehenden und längs deren Mittellinien verlaufenden Mittelebenen im wesentlichen parallel zueinander verlaufen, und daß die großen Basisflächen der trapezförmigen Einzelkörper (10, 11) im wesentlichen in einer zu den Mittelebenen senkrechten Ebene liegen, wobei die Einzelkörper (10,11 ) untereinander durch zwei elastische Verbindungselemente (12,13) miteinander verbunden sind, die senkrecht zu den Mittelebenen der Einzelkörper (10, 11 ) verlaufen und im Endbereich der großen Achse liegen, die eine gemeinsame Tangente der Sätze von Kugeln (8) bildet, die sich jeweils auf der Außenseite der Anordnung an den Eckpunkten von gleichschenkligen Dreiecken befinden, wobei diese Dreiecke entgegengesetzt orientiert sind.

7. Prothese nach Anspruch 6, **dadurch gekennzeichnet, daß** der aus mehreren Einzelkörpern bestehende Käfig (7) mit den Kugeln (8) ein Volumen aufweist, welches unter Berücksichtigung der Aufgabe des Käfigs als Lagerung der Kugeln möglichst gut dem Volumen des biologischen Nucleus angepaßt ist, so daß damit eine Selbstpositionierung der Prothese gesichert ist, durch die sich diese immer in der anatomischen Position befindet und die natürlichen Bewegungen zwischen zwei Wirbelkörpern wiederherstellt.

## Claims

1. Intervertebral nucleus prosthesis, the volume of which is adapted to that of the biological nucleus, **characterised in that** it consists of at least one spherical body (8), movable in two directions of a plane and made of a solid, non-oxidising, bio-compatible material, which is held as freely rotatable about its mid-point and indisplaceable in a cage (7) and which projects on both opposite sides of the cage in the form of a universal ball joint.

2. Prosthesis according to claim 1, **characterised in that** the opposite sides of the cage (7) are constructed as curved and are symmetrical in respect of a transverse centre plane.

3. Prosthesis according to one of claims 1 or 2, **characterised in that** the cage (7) comprises a housing (14) made of light, solid, non-oxidising, bio-compatible material, in particular of titanium, and contains a mass (15) made of a material with minimal friction coefficient, in particular polyethylene, and **in that** provided inside the mass is a receiving chamber for the movable spherical body (8), in which it is held captive as freely rotatable.

4. Prosthesis according to one of claims 1 to 3, **characterised in that** the cage (7) substantially has the shape of an isosceles trapezium and holds several identical balls (8), which in each case touch an identical fictitious plane lying outside the cage on both sides of the horizontal centre plane of the cage and which lie on both sides of the cage (7) at the corner points of an isosceles triangle.

5. Prosthesis according to one of the preceding claims, **characterised in that** the height of the universal ball joint projecting out of the cage amounts in each case to approximately a tenth of the diameter of the movable body (8).

6. Intervertebral nucleus prosthesis, the volume of which is adapted to that of the biological nucleus, **characterised in that** it has a cage (7), consisting of two identical individual bodies, wherein several spherical bodies (8) movable in two directions of a plane and made of a solid, non-oxidising, bio-compatible material, are held in each individual body as freely rotatable about their mid-point and indisplaceable and project on both opposite sides of the individual body (10) in the shape of a universal ball joint, towards which the two identical individual bodies (10, 11) are arranged in the shape of an isosceles trapezium and in such a way that their centre planes standing perpendicular on the parallel base faces and running along their centre lines run substantially parallel to one another, and **in that** the large base faces of the trapeze-shaped individual bodies (10, 11) lie substantially in a plane perpendicular to the centre planes, wherein the individual bodies (10, 11) are mutually connected to one another by two elastic connecting elements (12, 13), running perpendicular to the centre planes of the individual bodies (10, 11) and lying in the end region of the large axis, which forms a common tangent of the sets of balls (8) located in each case on the outside of the arrangement at the corner points of isosceles triangles, these triangles being orientated in opposite directions.

7. Prosthesis according to claim 6, **characterised in that** the cage (7), consisting of several individual bodies, with the balls (8) has a volume which, taking into account the object of the cage as bearing for the balls, is adapted as well as possible to the volume of the biological nucleus, thus ensuring self-positioning of the prosthesis, owing to which it is always located in the anatomical position and restores the natural movements between two vertebral bodies.

## Revendications

1. Prothèse nucléaire intervertébrale, dont le volume est adapté à celui du nucleus biologique, **caractérisée en ce qu'**elle est constituée par au moins un corps (8) sphérique mobile dans deux directions d'un plan, en matériau rigide, inoxydable, biocompatible, qui est monté librement rotatif autour de son centre et de manière fixe dans une cage (7) et qui émerge sur les deux côtés face à face de la cage sous la forme d'une calotte sphérique.

2. Prothèse selon la revendication 1, **caractérisée en ce que** les côtés face à face de la cage (7) sont cintrés et sont symétriques par rapport à un plan médian transversal.

3. Prothèse selon la revendication 1 ou 2, **caractérisée en ce que** la cage (7) est formée par un corps (14) en matériau léger, résistant, non oxydable, biocompatible, en particulier en titane, et contient une masse (15) réalisée dans un matériau avec de très faibles coefficients de frottement, en particulier le polyéthylène, et **en ce qu'**un logement est prévu à l'intérieur de la masse pour recevoir le corps (8) sphérique mobile, qui est monté librement rotatif dans celui-ci.

4. Prothèse selon l'une quelconque des revendications 1 à 3, **caractérisée en ce que** la cage (7) présente sensiblement une forme trapézoïdale isocèle et contient plusieurs billes (8) identiques, qui, sur les deux côtés du plan médian horizontal de la cage, entrent chacune en contact avec un plan fictif situé en dehors de la cage et qui sont situés sur les deux côtés de la cage (7) au niveau des angles d'un triangle isocèle.

5. Prothèse selon l'une quelconque des revendications précédentes, **caractérisée en ce que** la hauteur de la calotte sphérique en saillie hors de la cage correspond pratiquement au dixième du diamètre du corps (8) mobile.

6. Prothèse nucléaire intervertébrale, dont le volume est adapté à celui du nucleus biologique, **caractérisée en ce qu'**elle comporte une cage (7), qui est formée par deux corps élémentaires identiques, plusieurs corps (8) sphériques, mobiles dans deux directions dans un plan, en matériau rigide, inoxydable, biocompatible, étant montés librement rotatifs autour de leur centre et de manière fixe dans chacun des corps élémentaires et émergeant des deux côtés face à face du corps élémentaire (10) sous la forme d'une calotte sphérique, alors que les deux corps élémentaires (10, 11) identiques sont conçus en forme de trapèze isocèle et sont agencés de telle sorte que leurs plans médians perpendiculaires à leurs bases parallèles et s'étendant le long de leurs axes médians, sont sensiblement parallèles l'un à l'autre et que les grandes bases des corps élémentaires (10, 11) en forme de trapèze sont situées sensiblement dans un plan perpendiculaire aux axes médians, les corps élémentaires (10, 11) étant assemblés entre eux par deux éléments d'assemblage (12, 13) élastiques, qui sont perpendiculaires aux plans médians des corps élémentaires (10, 11) et sont situés dans la zone d'extrémité du grand axe qui forme une tangente commune aux jeux des billes (8) situées chacune sur le côté extérieur de l'ensemble au niveau des angles de triangles isocèles, ces triangles étant orientés en sens opposé.

7. Prothèse selon la revendication 6, **caractérisée en ce que** la cage (7), formée par plusieurs corps élémentaires, présente avec les billes (8) un volume qui, en tenant compte de la fonction de la cage comme logement pour les sphères, est adapté d'aussi près que possible au volume du nucleus biologique, de manière à garantir un auto-positionnement de la prothèse lui permettant d'être toujours située dans la position anatomique et de restaurer les mouvements naturels entre deux vertèbres.
